# EUROPEAN PATENT SPECIFICATION

(11) **EP 4 397 330 B1**
(45) Date of publication and mention of the grant of the patent: **19.08.2026**
(21) Application number: 23460040.1
(22) Date of filing: 05.12.2023
(51) Int. Cl.: A61L 27/26, A61L 27/38, A61L 27/52, B33Y 70/00

(54) **HYDROGEL BASED ON CHITOSAN AND AGAROSE FOR USE AS BIO-INK.**
HYDROGEL AUF BASIS VON CHITOSAN UND AGAROSE ZUR VERWENDUNG ALS BIO-TINTE.
HYDROGEL À BASE DE CHITOSAN ET D'AGAROSE POUR UTILISATION COMME BIO-ENCRE.

(30) Priority: 03.01.2023 PL 44340323
(43) Date of publication of application: 10.07.2024
(73) Proprietor: Politechnika Gdanska, 80-233 Gdansk (PL); Gdanski Uniwersytet Medyczny, 80-210 Gdansk (PL)
(72) Inventor: Mania, Szymon, 83-031 Legowo (PL); Tylingo, Robert, 80-125 Gdansk (PL); Banach-Kopec, Adrianna, 82-110 Sztutowo (PL); Wawrzynowicz, Agata, 80-180 Gdansk (PL); Pikula, Michal, 80-170 Gdansk (PL); Deptula, Milena, 80-174 Gdansk (PL); Czerwiec, Katarzyna, 80-211 Gdansk (PL)
(74) Representative: Pawlowska-Bajerska, Justyna

(56) References cited:
- EP-B1- 2 920 240
- ZAMORA-MORA VANESSA ET AL: "Chitosan/agarose hydrogels: Cooperative properties and microfluidic preparation", CARBOHYDRATE POLYMERS, APPLIED SCIENCE PUBLISHERS , LTD BARKING, GB, vol. 111, 28 April 2014 (2014-04-28), pages 348 - 355, XP028882142, ISSN: 0144-8617, DOI: 10.1016/J.CARBPOL.2014.04.087
- BUTLER HALEY M. ET AL: "Investigation of rheology, printability, and biocompatibility of N,O-carboxymethyl chitosan and agarose bioinks for 3D bioprinting of neuron cells", MATERIALIA, vol. 18, 1 August 2021 (2021-08-01), pages 101169, XP093130195, ISSN: 2589-1529, DOI: 10.1016/j.mtla.2021.101169
- JONATHAN M ZUIDEMA ET AL: "Fabrication and characterization of tunable polysaccharide hydrogel blends for neural repair", ACTA BIOMATERIALIA, ELSEVIER, AMSTERDAM, NL, vol. 7, no. 4, 29 November 2010 (2010-11-29), pages 1634 - 1643, XP028366361, ISSN: 1742-7061, [retrieved on 20101201], DOI: 10.1016/J.ACTBIO.2010.11.039

## Description

The subject of the invention is a thermosensitive composite based on a chitosan and agarose hydrogel - chitosan-based hydrogel composite, which can take the form of a sol or a gel, it's use as a spatial objects, especially utilizing additive manufacturing technology, and a method for producing a composite that can become a bio-ink based on chitosan and agarose hydrogel. The selected composition of this composite determines the speed and temperature of the sol-gel phase transition - transformation. Thanks to the ability to thermally regulate this transformation, the chitosan and agarose hydrogel composite can be used in the form of a bio-ink for creating spatial objects using additive manufacturing technology, hence the new use of the bio-ink is also a subject of the invention.

The 3D objects created from this bio-ink can serve as a universal scaffold that meets the requirements necessary for the survival, proliferation, and differentiation of the cells housed in it. This invention is particularly applicable as a matrix for cell culture in the production of food, medical, and cosmetic products.

The term "hydrogels" refers to materials based on polymers that possess a three-dimensional network structure comprising a water phase between the chains, where covalent or non-covalent interactions can occur.
doi: 10.1080/03602559.2011.593082

Hydrogels, especially formed from naturally derived polymers, constitute a group of interesting compounds whose application potential is determined by their physicochemical and biological characteristics. Stimuli-responsive hydrogels (sensitive to pH, temperature, ionic strength, electric or magnetic fields, light, and/or chemical and biological stimuli) can be used in drug delivery systems, allowing control over the rate of drug release, or as cellular carriers for tissue regeneration.

Most attention in the scientific literature has been devoted to systems in relation to temperature so far. There are two main types of thermosensitive polymers; the first exhibits a lower critical solution temperature (LCST), while the second exhibits an upper critical solution temperature (UCST). LCST and UCST are the respective critical temperature points below and above which the polymer and solvent are completely miscible.
doi: 10.3390/polym3031215.

In tissue engineering, thermosensitive polymers are commonly used in two situations: as substrates that enable cell growth and proliferation, and as injectable gels for *in situ* scaffold formation. In the first application, the thermoresponsive ability of polymers is utilized to regulate the attachment and detachment of cells from surfaces
doi: 10.1007/s10439-010-0035-1.

The second application involves the encapsulation of cells within three-dimensional structures.
doi: 10.3390/polym3031215.

Creating an *in situ* cell/scaffold construct, as opposed to *in vitro* formation, allows for the delivery of cells, nutrients, and growth factors to scaffolds of any shape using minimally invasive techniques. In the *in situ* method, a thermoresponsive polymer is mixed at room temperature with cells, then injected into the body. Upon injection, due to a temperature increase to 37°C, which is above the polymer's LCST, the sol transforms into a gel, encapsulating the cells within a three-dimensional structure of the hydrogel solid. With the advancement in additive manufacturing technology, materials science, and stem cell biology, 3D bioprinting has enabled the generation of functional tissues *in vitro.*
doi: 10.1038/nbt.3413, doi: 10.15259/pcacd.25.003

However, materials used in bioprinting must meet criteria such as biodegradability, biocompatibility, non-cytotoxicity, and porosity to provide the necessary conditions for new tissue formation and support cell growth during regeneration. Additionally, cellular scaffolds must be adhesive, allow for the exchange of gases and metabolites, and possess appropriate mechanical properties, especially in the case of polymer hydrogels. Hydrogels have a structure similar to natural body matrices. Moreover, due to their high porosity, hydrogels are capable of transporting low molecular weight substances and nutrients essential for cellular activity.
doi: 10.1080/00914037.2018.1562924.

The biggest challenge in creating materials and structures for devices capable of producing biocompatible 3D prints comprising cells lies in the rheology of hydrogels and the ability to control it while maintaining comfortable conditions for the cells. One of the natural polymers that meets most of these requirements is agarose, which plays a structural role. It does not carry biological activity.

Chitosan is a cationic polymer derived from chitin through N-deacetylation, consisting of β(1->4)-glucosamine and N-acetyl-D-glucosamine subunits. Due to its properties (non-toxicity, biocompatibility, biodegradability), it is used in many industries. The main technological drawback of this polymer is its lack of water solubility, related to the presence of strong hydrogen bonds within and between chitosan molecules. However, it is readily soluble in acid solutions. This limits the applications of chitosan materials, especially where it directly contacts bacterial or cell cultures. This limitation applies to both chitosan hydrogels and xerogel materials of this polymer obtained by sublimation drying. In both types of materials, the acid used to dissolve the polymer is toxic to cells and reduces the material's biocompatibility. This issue can be addressed by chemically modifying chitosan molecules to produce water-soluble polymers or by fragmenting the chitosan chain to obtain water-soluble chitosan oligosaccharides. Many methods for this procedure are known in scientific and patent literature.

From the publication by Liu and colleagues (2021), a bio-ink used in the reconstruction of nerve tissue, including the repair of spinal cord injuries, is known. To improve cell viability and minimize interactions between cells and the material, a bio-ink was proposed consisting of functional chitosan (hydroxypropyl chitosan- HBC, thiolated hyaluronic acid - HA-SH, vinyl sulfonated hyaluronic acid- HA-VS, and Matrigel - MA). The bio-ink composition of 3% HBC, 0.6% HA-SH, 0.6% HA-VS, and 0.1% MA was characterized by a rapid gelation time of 20 seconds and the ability to spontaneously form covalent bonds. Complete crosslinking of the bio-ink occurred at 37°C after a 2-hour incubation. Bio-inks containing neural stem cells were used in 3D printing by maintaining the syringe temperature at 10°C and the worktable temperature at 37°C.
doi:10.1016/j.biomaterials.2021.120771

Maturavongsadit and colleagues (2021) proposed a method for creating a bio-ink for bone tissue regeneration based on nanocellulose and 3% chitosan. The solution of the problem of biocompatible bio-inks for specific tissue structures involves the use of thermogelling chitosan, glycerophosphate, hydroxyethyl cellulose, and cellulose nanocrystals (CNC). By varying the concentrations of glycerophosphate (80-300mM), hydroxyethyl cellulose (0-0.5mg/mL), and CNC (0-2% m/V), it is possible to promote rapid gelation kinetics, i.e., below 7 seconds at 37°C, while maintaining the integrity of the 3D print shape. The bio-ink containing MC3T3-E11 osteoblast cells was printed while maintaining the extruder nozzle temperature at 25°C and the worktable temperature at 37°C.
doi: 10.1021/acsabm.0c01108

Ku and others (2020) proposed a thermosensitive bio-ink for bioprinting applications. The chitosan hydrogel solution was prepared by dissolving it in 0.1M acetic acid. As crosslinking agents, 56% β-glycerophosphate, 8% potassium phosphate, and 3.5% sodium bicarbonate were used, which were prepared in the culture medium. The chitosan solution was mixed with the crosslinking agents in a 9:1 ratio. After mixing, the solutions were adjusted to a pH of 6.5 using a 1M NaOH solution. The printing problem was solved by using polycaprolactone as a structural element, onto which layers of thermosetting bio-ink at 37°C were applied. In the bio-ink tests, human periodontal ligament stem cells (PDLSC) were used.
doi: 10.3390/app10072455

Hafezi and colleagues (2020) proposed a method for creating a highly repeatable bio-ink for skin regeneration, which involves crosslinking chitosan with genipin. The preparation involved making a 1.2% chitosan and 1.2% PEG solution in 0.25% acetic acid, followed by adding 5mL of a 1% genipin solution. The hydrogel was autoclaved and then mixed at a 5:1 ratio with keratinocytes and skin fibroblasts suspended in DMEM. Additionally, a 6% sodium alginate solution was prepared. The bioprinting problem was solved by first printing a layer of sodium alginate, then crosslinking it with 5% CaCl2, followed by subsequent layers of chitosan-genipin-PEG. Bioprinting was conducted at 37°C.
doi: 10.3390/pharmaceutics12060550

From the publication by Tonda-Turo and colleagues (2020), a method for creating a photo-curable chitosan bio-ink containing NIH/3T3 fibroblasts, Saos-2 osteoblasts, and Sh-SY5Y neuroblasts is known. The crosslinking problem was solved by giving the chitosan a thermally induced phase transition. Methacrylated chitosan was dissolved in water at a concentration of 1.5%, mixed overnight, and stored at 4°C. The hydrated disodium salt of β-glycerophosphate was prepared in culture medium, which was then dropped into the methacrylated chitosan solution. A 0.05% lithium phenyl-2,4,6-trimethylbenzoylphosphinate was used as a photoinitiator and added to the mixture. Bioprinting involved maintaining the bio-ink at 4°C, which then crosslinked after being extruded onto a worktable at 37°C.
doi: 10.1016/j.bprint.2020.e00082

From the publication by Zou and others (2021), a method for creating hydrogel composites based on agarose, nanocellulose, and sodium alginate used for facial reconstruction is known. The bio-ink was obtained by mixing solutions of 1% sodium alginate, 0.5% nanocellulose, and 0.5-2% agarose. In the proposed printing method, the printed composite discs were additionally crosslinked with a 2% CaCl2 solution. The problem of hydrogels collapsing during printing was solved by printing support elements using poly(vinyl alcohol). Human umbilical vein endothelial cells (HUVECs) were used in the tests. The printing temperature was 37°C.
doi: 10.1016/j.carbpol.2021.118222

Gu and colleagues (2020) proposed a method for creating a bio-ink composed of 8% agarose (0.2% native agarose and 7.8% carboxylated agarose). The use of carboxylated agarose aimed to increase the amount of β-sheet and β-strand structures in the polymer skeleton while reducing α-helices, thus lowering the sol-gel transition temperature to physiological or below. The proposed printing method involved heating the bio-ink to 90°C, then cooling it to 42°C for 10 minutes. After adding human nasal chondrocytes to the bio-ink and placing it in a cartridge, printing was conducted at 37°C.
doi: 10.3390/bioengineering7040141

From the publication by Duarte Campos and colleagues (2019), a method for creating miniaturized 3D *in vitro* cancer models using bio-inks based on agarose and type I collagen is known. The 3% agarose solution was prepared by dissolving the polymer and autoclaving at 121°C for 15 minutes. Meanwhile, a type I collagen hydrogel was obtained by mixing eight parts of 0.4% acidic collagen solution with one part of 10x DMEM and one part of cell suspension in the appropriate medium. The 3D printing bio-ink was obtained by mixing 3% agarose with 0.3% type I collagen at final concentrations of 0.5% and 0.2%, respectively. The bio-inks were loaded with cells: human primary mesenchymal stromal cells from the umbilical cord (UC-MSC), human primary umbilical vein endothelial cells (HUVEC), and epithelial-neuroblastoma cells (SH-SY5Y).
doi: 10.3390/cancers11020180

Seidel and colleagues (2017) proposed a bio-ink based on alginate, agarose, and methylcellulose for forming three-dimensional matrices with a specific internal pore architecture, intended for plant cells. In this method, the bio-ink consisted of 2.8% alginate by mass, 0.9% agarose, and 2% methylcellulose. Initially, alginate and agarose were dissolved in water and autoclaved at 121°C for 20 minutes. Methylcellulose was then added at room temperature to the alginate and agarose solution, followed by incubation for 2 hours. The bio-ink was extrusion printed, and the printed scaffold was further crosslinked by incubation in a 0.1M CaCl₂ solution containing 3% sucrose for 10 minutes. For bioprinting plant cells, an in vitro basil cell culture (*Ocimum basilicum L. var. purpurascens Benth.* 'Cinnamon Basil') was used, which was added to the bio-ink just before printing.
doi: 10.1088/1758-5090/aa8854

The bio-ink for printing functional 3D mini neural tissues was described in a publication by Gu and colleagues (2016). The proposed bio-ink consisted of 5% alginate, 5% carboxymethylated chitosan, 1.5% agarose, and neural stem cells. The bioprinting problem was solved by using a worktable temperature of 15°C and additional crosslinking by immersing the printed scaffold in 2% CaCl2 for 10 minutes.
doi: 10.1002/adhm.201600095

From the patent description CA 2442868, a method for creating matrices based on chitosan and hydroxycarboxylic acids for use in 3D printing is known. In this solution, chitosan is dissolved in a selected hydroxycarboxylic acid, such as hydroxyacetic acid, dried at 50°C until a film forms, and then neutralized with a sodium hydroxide solution.

From the description in CN101148520B, a method for creating a temperature-sensitive chitosan hydrogel containing 0.2-20% wt. chitosan, 1-60% wt. glycerin or 0.5-40% wt. mannitol, and 0.5-20% wt. borax is known. The hydrogel is prepared by dissolving chitosan in an acid solution, adding a solution of glycerin or mannitol, then adding a borax solution with mixing and heating to the gelation temperature to obtain the hydrogel. This temperature-sensitive chitosan hydrogel is liquid at a lower temperature, becomes gel at body temperature, and can be used as an injectable implant material and for 3D printing.

The US20060127873A1 describes a method for producing hydrogels dedicated to tissue repair, characterized by cytocompatibility and self-gelling ability. The composition includes a biocompatible mixture of chitosan, a bifunctional dialdehyde, and a hydroxylated polymer, which can be used for immobilization or encapsulation of living cells or bioactive substances. The method involves mixing bioactive substances, living cells, and/or components of the extracellular matrix with a cross-linking solution containing a bifunctional hydroxylated polymer treated with an aldehyde, such as hydroxyethyl cellulose. The cross-linking solution is then homogeneously mixed with a neutral isotonic chitosan solution. The chitosan is cross-linked by the bifunctional aldehyde, while the cells are protected from the potentially harmful effects of the aldehyde cross-linking agent by the hydroxylated polymer. The injectable solution maintains the viability and bioactivity of cells and immobilizes them at the injection or delivery site. Depending on the specific application, mixtures of chitosan and bifunctional dialdehyde can be used. The injectable solution also releases bioactive substances with controlled release kinetics from the injection site.

The US20170143831A1 patent presents a method for producing bio-ink for *in vivo* bone tissue repair using methacrylated gelatin or methacrylated chitosan with nanoscale silicate-based compounds (laponite) and viscosity regulating agents, including sucrose, as well as a photoinitiator in the form of 2-hydroxy-1-(4-(hydroxyethoxy)phenyl)-2-methyl-1-propanone. This raw material mixture is susceptible to hardening under UV light, which is analogous to the operation of SLA (Stereolithography) printers.

From the description in the US20170306295A patent, there is an invention known for obtaining an injectable gel for the repair of articular cartilage tissues, containing chitosan hydrogel particles and cells capable of forming articular cartilage. This method includes a stage of primary cell amplification in a three-dimensional structure containing particles of physical chitosan hydrogel or a chitosan derivative. This is followed by a stage of redifferentiation and induction of extracellular matrix synthesis by the aforementioned amplified cells, in the same three-dimensional structure, which constitutes the main advantage of the invention. The method of processing chitosan to obtain a polymer hydrogel is based on standard methods of dissolving the polymer in diluted acid solutions, doping with propylene glycol, and conditioning at an elevated temperature (50°C) for several to several dozen hours.

From the description in the WO2016209062A1 patent, there is a known method for producing bio-ink based on two components in the form of solutions, where both consist of mixtures of groups of biopolymeric compounds. The first mixture contains a biopolymer with a first functional group and a biopolymer with a net negative charge (carboxymethyl cellulose, hyaluronic acid, β-glycerophosphate). The second mixture contains a biopolymer with a second functional group that reacts with the functional group of the compound from the first mixture, as well as a biopolymer with a positive charge (chitosan or N-isopropylacrylamide). This allows for the occurrence of electrostatic interaction between biopolymers with opposite charges - the formation of polyelectrolyte complexes.

A similar invention to the presented method for producing bio-ink based on chitosan and agarose is proposed in the WO2017135498A1 patent description. The method involves preparing a hydrogel based on chitosan and nucleic acid. According to the invention, chitosan is dissolved in diluted acetic acid, nucleic acid in a buffer solution of disodium phosphate dodecahydrate, and then the solutions are combined and mixed at 60°C for an hour, followed by cooling to room temperature. The mass ratio of nucleic acid to chitosan ranges from 20:1 to 10000:1.

The most closely related solution to the presented method for producing bio-ink based on chitosan and agarose was proposed in a publication by Butler and co-authors (2021), which requires the use of a chitosan derivative to ensure its solubility in water in a neutral environment. The bio-ink consists of N,O-carboxymethylchitosan and agarose with an optimal polymer ratio of 60% and 40%, respectively. In this solution, chitosan, 10N sodium hydroxide, isopropanol, and deionized water are mixed for 2.5 hours at room temperature. Then, a mixture of monochloroacetic acid and isopropanol is added dropwise for 30 minutes and stirred for 4 hours at 50°C. The solid is separated from the solution, washed with 90% ethyl alcohol, and dried at 40°C. The prepared N,O-carboxymethylchitosan is dissolved in water to make its concentration 10%. A 1% agarose solution is obtained by suspending it in water and autoclaving. To obtain bioprinting ink, agarose is kept at 100°C, and then added to the N,O-carboxymethylchitosan solution in a 4:6 ratio. The mixture is maintained at 37°C to prevent agarose solidification. The bioprinting issue is solved by using a heated printhead at 37°C. Bioprinting ink laden with neuro2A neuronal cells was printed using a pneumatic bioprinter. However, this method requires the use of a water-soluble chitosan derivative.
doi: 10.1016/j.mtla.2021.101169

In all the known solutions - above-mentioned works, the appearance of chitosan as one of the components of the bioprinting ink is associated with the need to dissolve the polymer in an acid solution, which in one of the subsequent steps is neutralized. These methods also foresee the use of water-soluble chitosan derivatives and/or the use of auxiliary polymers in the form of sodium alginate crosslinked with calcium chloride. All of the above solutions include a step of increasing the temperature to induce the sol-gel transition of the bioprinting ink.

A drawback of known solutions is the presence of acid in hydrogel chitosan solutions used for combining it with other components, including agarose. The presence of acid in the composite system is a key limitation, as it prevents the gelation of the composite due to the presence of agarose, significantly prolongs its gelation time, or allows for the formation of gels with very poor mechanical properties. Due to these limitations, the use of a hydrogel chitosan-agarose composite in additive manufacturing technology is impossible, as it does not allow for the rapid formation of successive layers of a two-dimensional object. Techniques for removing acid residues from chitosan materials are known. However, these involve extracting acid from finished materials or neutralizing it in finished materials, which is not feasible in additive manufacturing technology.

The goal - objective of the invention was to provide a chitosan-based composite that can take the required form of bioprinting ink under favorable conditions, especially temperature conditions, through a sol-gel phase transition - changing phase - for use in spatial - 3D prints as cell cultures after placing living cells/cell lines in it. It is also possible to add other biologically active compounds to the cell culture and, through thermal induction, organize the polymer network due to the obtained properties, with the aim of its dedicated use for creating spatial objects for cell cultures.

Agarose is a biocompatible polysaccharide that has been shown to be usable as a three-dimensional scaffold in tissue engineering. In its hydrogel form, agarose has a porous structure and provides a conducive environment for the spread and proliferation of cells. However, agarose alone lacks the ability to increase cell adhesion, which somewhat limits its application. Chemical functionalization of agarose can improve the adhesion of cultured cells, but the complex preparation process is costly and time-consuming. Another issue with chemical functionalization is the introduction of toxic organic solvents, which could be potentially harmful for the subsequent growth of cells. To maximize the use of agarose without a complex chemical process, in accordance with the essence of the invention, the focus was on developing a composite that is free of the aforementioned drawbacks and dedicated to use in cell cultures. On the other hand, the focus was on mixing agarose and chitosan hydrogels, achieving the desired characteristics of both polymers for cell cultures. Chitosan proved to be both safe in cell culture and supportive of cell adhesion to the scaffold surface, as well as their proliferation and differentiation. Moreover, this polymer has antimicrobial activity, so the bioprinting ink will have a microbiological protection function.

in comparison to the known solutions, the invention differs in composition - ingredients of the composite, manufacturing procedure - steps and parameters, and there Is differ use of the composite - the way and the form of this use - the use of the composite is in the form of bioprinting ink since it is characterized - enable to perform the phase transition from sol to gel that occurs during the cooling of the composite with a temperature gradient ranging from 5 to 39°C, especially 5-36°C, preferably 28-36°C. Importantly, heating the resulting bioprinting ink already in gel form above 36°C up to 50°C, with relative air humidity above 80%, does not cause a reverse transformation (gel-to-sol). Therefore, the transition from sol to gel form enables the creation of bioprinting ink and from it three-dimensional cell cultures comprising cells/cell lines. This allows for the preservation of the structure of spatial objects, especially with the use of additive manufacturing technology - three-dimensional objects created based on the developed bioprinting ink, thereby confirming the possibility of using the invention for this purpose.

The method for producing a hydrogel composition - a composite taking the form of bioprinting ink after a sol-gel phase transition comprises several steps. The method involves initially dissolving chitosan in a solution of hydroxyoctanoic acid or another volatile organic acid with a pKa in the range of 3.0 to 5.0, at a mass concentration of 0.5% to 1.5% w/w relative to the acid solution, in an amount ensuring a polymer concentration in the range of 1.0% to 2.5% w/w relative to the acid solution. The hydrogel has a pH of 6.0-6.5 and is produced using a polymer with a molecular weight in the range of 50-375 kDa, either low (50-180 kDa), medium, or high (300-375 kDa), most advantageously of medium molecular weight (180-300 kDa) and a degree of deacetylation in the range of 60-95%, preferably above 75%. In the next step, chitosan is precipitated from the previously prepared solution into a microcrystalline precipitate. The chitosan precipitate is washed three times with distilled water, which constitutes two to three times the mass of the preliminarily separated precipitate. The deposit is then suspended in distilled water in an amount ensuring a polymer concentration in the range of 0.5% to 1.5% w/w relative to the mass of the suspension, and after homogenization, it is subjected to carbon dioxide saturation in the amount of 0.1 - 1L/L relative to the homogenized suspension. The resulting chitosan hydrogel with pH=6.0-6.5 is mixed with agarose hydrogel, in a mass share ranging from 20 to 80% relative to the agarose hydrogel, wherein the agarose hydrogel obtained from the polymer has a strength of 1.5% gel of at least 800 g/cm2 and a gelation temperature < 40°C, and preferably has a low electrophoretic mobility value (EEO <0.15), and wherein the agarose hydrogel is previously obtained by dissolving this polymer in distilled water at a temperature range of 60-80°C in an amount ensuring a concentration of agarose in the range of 0.5 - 1.5% w/v and additional cell lines and/or cells for culture are added in an amount of 1-10% w/w relative to the composite.

The uniform composite obtained after mixing both hydrogels chitosan-agarose is cooled to a temperature of 40-45°C. To use the obtained composite in the form of bioprinting ink for obtaining spatial objects, especially using additive manufacturing technology - 3D forms, especially cell cultures - to obtain a 3D form - application of the composite, the above-described composition of the composite mixture is supplemented with an addition of cells/cell lines in the form of a suspension, sediment, solution, or dry substance, to obtain bioprinting ink.

Once the printing process begins - to achieve the gel formation from sol, the temperature of the composite obtained according to the invention, the bioprinting ink, is lowered due to its extrusion onto the working area of the printer. This initiates the sol-gel phase transition during cooling with a temperature gradient of the phase transition ranging from 5 to 39°C, especially 5-36°C, advantageously in the range of 28-36°C. The transformation occurs quickly enough to utilize this property in creating successive two-dimensional layers of 3D objects.

The invention also sets the product obtained by this method, namely the composite based on chitosan and agarose hydrogels that can exist in either a sol or gel form. This composite can be used as a form of bioprinting ink for producing 3D prints, preferably after the addition of cell lines/cells through a phase transition in the aforementioned temperature range. In this state, the gel enables the creation of spatial objects, especially using additive manufacturing technology - 3D printing, preferably for cell culture. The invention sets the use of aforementioned.

The composite obtained in this way, in both sol and gel forms, comprises the following composition:

| **Ingredient** | **Content [% m/m]** |
|---|---|
| Water | 98,9 - 98,99 |
| Chitosan | 0,2 - 0,8 |
| Agarose | 0,2 - 0,8 |
| Carbon dioxide | 0,01 - 0,1 |

Agarose and chitosan, for the purpose of obtaining the composite, are added in the form of hydrogels comprising water. However, the content in the product is calculated separately for water and for chitosan/agarose.

The composite is produced based on chitosan hydrogel with a pH of 6.0-6.5, in which this polymer has a molecular weight ranging from 50-375 kDa, including low (50-180 kDa), medium, or high (300-375 kDa), most advantageously medium molecular weight (180-300 kDa) and a degree of deacetylation in the range of 60-95%, preferably above 75%, and agarose hydrogel obtained from the polymer, advantageously with a low electrophoretic mobility value (EEO <0.15), it has a strength of 1.5% gel of at least 800 g/cm² and a gelation temperature < 40°C. The mass share of chitosan in the composite ranges from 0.2% to 0.8% relative to the mass of the composite, advantageously 0.4%, while that of agarose ranges from 0.8% to 0.2% relative to the mass of the composite, advantageously from 0.6%. The remaining components of the composite consist of carbon dioxide in a mass share of 0.01% to 0.1% relative to the mass of the composite, advantageously 0.05%, and water. It additionally comprises cells for culture and/or cell lines in culture medium in the form of a suspension in a ratio of 1-10% w/w relative to the mass of the composite. The polymer mixture in the composite advantageously constitutes 1%. The composite, after the addition of elements such as for cell culture needs - cells for culture / cell lines in the form of suspension, precipitate, solution, or dry substance, advantageously in a mass ratio of 1:99 to 10:90 w/w, takes the form of ready-to-use bioprinting ink. The composite according to the invention is characterized in that it has the form of a sol or a gel and thus takes the form of bioprinting ink depending on the temperature of its treatment - i.e., in the range of 40-45°C it is a sol, and in the range of 5-39°C, especially 5-36°C, a sol-gel phase transition occurs, which enables the production of a 3D print from the bioprinting ink.

The invention also encompasses the use of the aforementioned composite in the form of bioprinting ink for creating spatial objects - 3D. To obtain an object from the bioprinting ink - a print, its transition from the sol form to the gel form is utilized, depending on the temperature treatment - i.e., in the range of 40-45°C, it is a sol, and in the range of 5-39°C, especially up to 36°C, a sol-gel phase transition occurs. After undergoing the sol-gel transition, the sol form does not revert back, thus realizing the use of the composite as bioprinting ink for the purpose of spatial printing from the composite.

The antimicrobial activity of the composite and bioprinting ink form based on chitosan, agarose, carbon dioxide, and water allows for the reduction of harmful bacteria *S. aureus* and *E. coli,* which infect cell lines present in the bioprinting ink, by 1.8-2.0 and 2.7-2.9 logarithmic orders, respectively, when the chitosan content constitutes between 0.4% and 0.6% of the composite mass - this range is described in examples - research results, which also suggest antimicrobial activity at 0.2 - 0.8%. The chitosan, agarose, carbon dioxide, and water-based composite does not show cytotoxic effects across the entire range of component shares relative to 46BR.1N fibroblasts and HaCaT keratinocytes. Moreover, across the entire range of component shares, it demonstrates a pro-proliferative effect relative to the 46BR.1N fibroblast cell line.

The invention is further illustrated in examples and a drawing, where on each figure is shown:
Fig. 1: A 3D hydrogel print made using the bioprinting ink according to Example 2.
Fig. 2: A 3D xerogel print made using the bioprinting ink as described in Example 3.
Fig. 3: Viscometric measurement of the sol-gel transition of the chitosan-agarose composite comprising 20, 40, 60, 80, 100% agarose (AG).
Fig. 4: Antimicrobial activity of the chitosan-agarose bioprinting ink. From left: 0, 40, 60, 100% agarose (AG) content (n=3, p<0.05).
Fig. 5: Assessment of the effect of bioprinting ink on the proliferation of HaCaT and 46BR.1N cells (n=3, p<0.05).
Fig. 6: Evaluation of the cytotoxicity of bioprinting ink against HaCaT and 46BR.1N cells (n=3, p<0.05).

### Example 1: Method of obtaining hydrogel composite and its application in the form of bioprinting ink for obtaining cell lines.

Chitosan with a molecular weight of 250 kDa and a degree of deacetylation of 75% was dissolved in 0.1 M hydroxyoctanoic acid at a concentration of 1.5% (w/w) and mixed using a mechanical stirrer at a rotational speed of 200 RPM until a homogeneous solution without undissolved solids was obtained. Then, while stirring, a 0.5M sodium hydroxide solution was gradually added to the chitosan solution until the pH of the solution reached 9. The resulting chitosan precipitate was separated under reduced pressure. The separated precipitate was alternately washed with distilled water and drained until the pH of the washing water reached 7. The washed and separated precipitate was supplemented with distilled water in such a quantity that, in terms of the mass of the dehydrated polymer (before precipitation), a 1% w/w aqueous solution was obtained. This chitosan precipitate suspension was then homogenized for 1 minute at a rotational speed of 10000 RPM, followed by gaseous saturation with carbon dioxide at a gas flow rate of 25 mL/min in an open container while simultaneously stirring mechanically at a rotational speed of 300 RPM until a homogeneous and clear solution was obtained, which corresponds to the dissolution of chitosan at the concentration of carbon dioxide. The pH of the chitosan solution thus obtained was 6.4.

In the next step, a 1% solution of agarose with a low electrophoretic mobility value (EEO <0.15), a strength of 1.5% gel equal to 1000 g/cm², and a gelation temperature <40°C was prepared. Agarose was gradually added to water at a temperature of 80°C, stirred at a rotational speed of 200 RPM, and the mixing was maintained until the polymer was completely dissolved. After adding the last portion of agarose, the vessel with the solution was closed to limit water evaporation. The uniform agarose solution was then cooled to a temperature of 70°C. The chitosan and agarose hydrogel composite was prepared by mechanically mixing these components in a 1:1 mass ratio using mechanical stirring at 200 RPM at a temperature of 45°C. In this way, a composite according to the invention was obtained, which can be used in the form of bioprinting ink for producing 3D prints. Its practical application in obtaining ready-to-use bioprinting ink - i.e., a composite with cell lines - is described further.

Subsequently, cells in the form of a suspension in culture medium were added in a 1:9 ratio to the mass of the hydrogel composite of chitosan and agarose, obtaining a ready-to-use bioprinting ink comprising living cells at a concentration of 10⁴-10⁸ cells per mL of bioprinting ink, preferably 10⁶ cells/mL of ink. The bioprinting ink was then used to create a 3D print, during which its temperature was lowered while applying successive 2D layers, down to 20°C, inducing the sol-gel phase transition.

### Example 2: Use of the obtained bioprinting ink in cell culture.

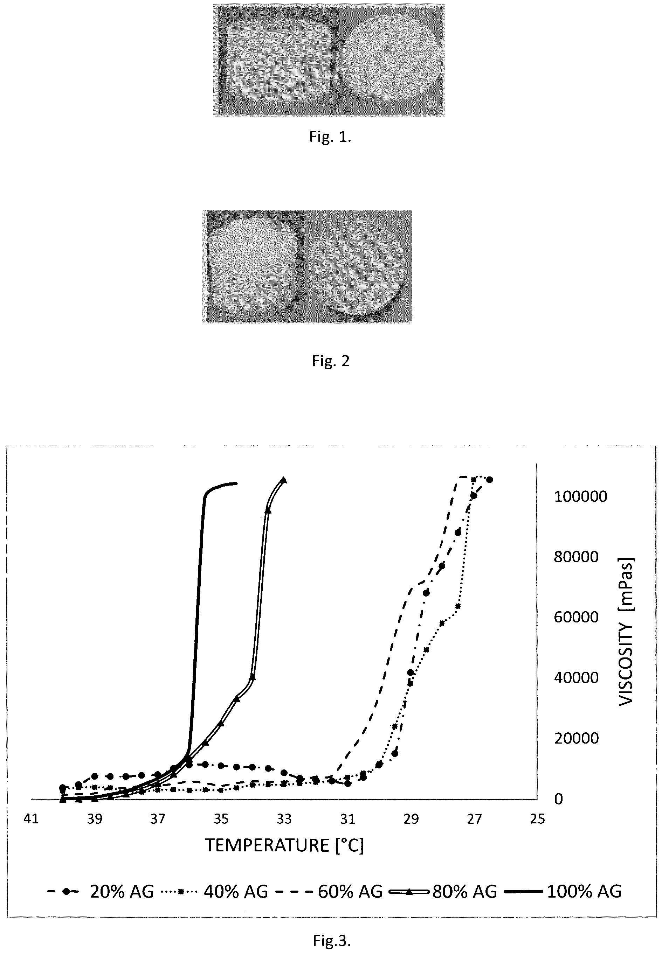

For the hydrogel composite in liquid form prepared in the amount of 90 g according to Example 1 at a temperature of 45°C, 10 g of a suspension of HaCaT keratinocytes and 46BR.1N fibroblasts comprising 3 x 10⁷ of both types of cells/g of suspension was added. The entire procedure was carried out under sterile working conditions, and the added cell suspension was mixed with the composite at 40°C and stored at this temperature until used in 3D printing. Fig 1 shows a 3D print made based on the bioprinting ink as per the above description, which can be used in creating skin-like implants, skin-like dressings, or skin research models. This confirms the sol-gel transformation and the achievement of mechanically stable, hydrated structures capable of maintaining shape in conditions of relative air humidity, in accordance with the description of the invention.

### Example 3. Preparing a dried form of bio-ink

A 3D print was prepared according to Example 2 and subjected to freeze-drying to obtain a cellular scaffold with cells in a dehydrated form. The print was frozen at -80°C for a period of 12 hours, and then freeze-dried at a pressure of 0.94 mbar, a condenser temperature of -80°C, and a temperature gradient of 100°C, over a period of 72 hours. Figure 2 shows freeze-dried 3D prints comprising HaCaT keratinocyte and 46BR.1N fibroblast cell lines, characterized by mechanical stability, high porosity and absorbency, and the ability to maintain shape.

### Example 4. Confirmation of the Sol-Gel phase transition

The sol-gel phase transition was measured for a chitosan-agarose hydrogel composite obtained according to Example 1. The test was conducted using a Brookfield DV-III ULTRA viscometer at a shear rate of 1.1 s⁻¹ (LV SC4-25) in the temperature range of 40-25°C with a temperature drop of 1°C every 10 minutes. As shown in Fig. 3, during the temperature decrease, the occurrence of the sol-gel phase transition was registered in the temperature range of 36-28°C, and a decrease in the phase transition temperature with an increase in the chitosan content in the bioprinting ink was observed. This confirms the feasibility of using the bioprinting ink in 3D printing technology. A steep slope of the curves at the phase transition temperature is observed, indicating a short transformation time required to create successive two-dimensional layers.

### Example 5. Confirmation of antimicrobial properties

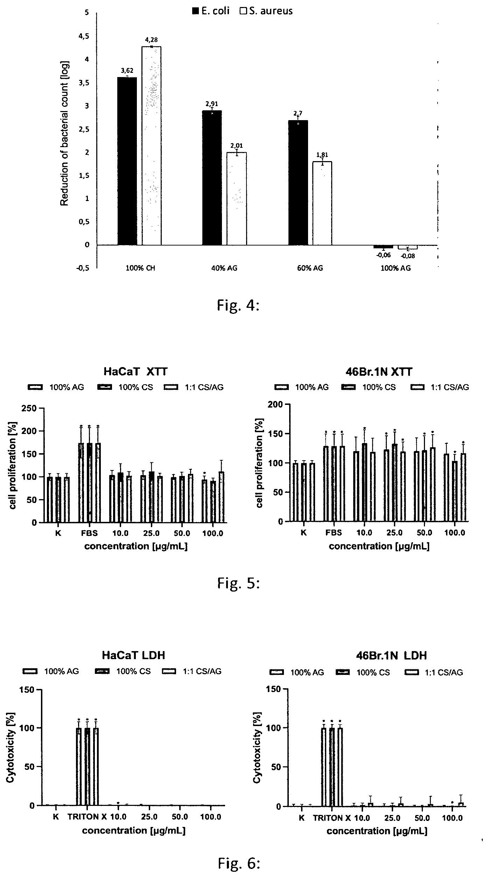

The antimicrobial activity was tested for materials prepared according to Example 3 based on the ASTM E2149 test against strains of bacteria *Escherichia coli* K-12 PCM 2560 (NCTC 10538) and *Staphylococcus aureus* PCM 2054 (ATCC 25923). The results of the antimicrobial activity are presented in Figure 4 as the logarithmic degree of microbial reduction as a function of the polymer share in the lyophilized 3D print. As shown in Fig. 4, the antimicrobial activity decreases with an increase in the agarose content in the bioprinting ink and is dependent on the type of bacterial strain. Gram-positive bacteria, exemplified by *S. aureus,* show greater sensitivity to the action of chitosan compared to the *E. coli* strain, which represents Gram-negative bacteria, as confirmed by the results presented for the material made exclusively from this polymer. In chitosan-agarose composites, this sensitivity is reversed concerning the considered strains, which may result from the availability of chitosan in the composites and their spatial structure. The antimicrobial activity of the bioprinting ink based on the chitosan-agarose composite allows for a reduction of *S. aureus* and *E. coli* bacteria by 1.8 - 2.0 and 2.7 - 2.9 logarithmic orders, respectively, when its share constitutes between 40 and 60% of the composite mass. This means that the bioprinting ink has at least bacteriostatic activity, which constitutes a function of microbiological protection of the bioprinting ink.

### Example 6. Confirmation of non-cytotoxicity and proliferation ability of skin cells

Biotprinting ink in lyophilized form was prepared according to Example 3. The obtained materials were flooded with DMEM HG culture medium with antibiotics (50 units/mL penicillin and 50 µg/mL streptomycin) - 25 mg of bioprinting ink per 1 ml of medium and incubated under standard conditions at 37°C, 5% CO2 for 24 hours. Subsequently, the medium was collected, and cells (HaCaT keratinocytes and 46BR.1N fibroblasts) were stimulated with the prepared extract. In addition to 100% extract, dilutions at concentrations of 10, 25, and 50% were also prepared. The cells were incubated for 24 hours, after which an LDH cytotoxicity test and an XTT proliferation assay were performed. As shown in Fig. 5, the bioprinting ink based on the composite of chitosan and agarose hydrogels does not show cytotoxic effects across the entire range of both components against 46BR.1N fibroblasts and HaCaT keratinocytes. Moreover, across the entire range of both components, it demonstrates a pro-proliferative effect relative to the 46BR.1N fibroblast cell line.

## Claims

1. Spatial objects for use in cell cultures and/or with cell lines, wherein the spatial objects are obtained by bioprinting an ink in a form of hydrogel composite , **characterized in** the ink has the form of a sol or a gel during the sol-gel phase transition, wherein the composite comprises water, chitosan in mass share of chitosan of 0.2-0.8% w/w, preferably 0.4% w/w, agarose in an amount of 0.2-0.8%, preferably from 0.6%, relative to the mass of the composite, and carbon dioxide in a mass share of 0.01-0.1% relative to the mass of the four-component composite, preferably 0.05%, and chitosan hydrogel has a pH of 6.0-6.5, wherein the polymer has a molecular weight in the range of 50-375 kDa and a degree of deacetylation in the range of 60-95%, preferably above 75%, and an agarose hydrogel obtained from this polymer has a strength of 1.5% gel of at least 800 g/cm² and a gelation temperature < 40°C, and preferably has a low electrophoretic mobility value (EEO <0.15), and it additionally comprises cells for culture and/or cell lines in the form of a suspension in a ratio of 1-10% w/w relative to the mass of the composite.

2. Spatial objects for use in cell cultures and/or with cell lines according to claim 1, wherein, in the range of 40-45°C it is a sol, and in the range of 5-39°C, preferably in the range of 5-36°C, a sol-gel phase transition occurs that enables the creation of cell cultures and/or cell lines from the composite bioprinting ink.

3. Method of manufacturing cell cultures and/or cell lines obtained from bioprinting ink in a form of hydrogel composite based on chitosan and agarose, **characterized in that** the method comprises step of initially dissolving chitosan in a solution of hydroxyoctanoic acid or another volatile organic acid, preferably with a pKa in the range of 3.0 to 5.0, in an amount ensuring a concentration of this polymer in the range of 1.0% to 2.5% w/w relative to the acid solution, while the chitosan has a molecular weight in the range of 50-375 kDa, preferably 180-300 kDa, and a degree of deacetylation in the range of 60-95%, and in the next step, chitosan is precipitated from the previously prepared solution into a microcrystalline deposit, and the chitosan deposit is washed with water, which constitutes at least twice the mass of the preliminarily separated deposit, and after that the deposit is suspended in distilled water in an amount enabling to obtain a polymer concentration in the range of 0.5% to 1.5% w/w relative to the mass of the suspension and, after homogenization, is subjected to saturation with carbon dioxide in the amount of 0.1 - 1L/L relative to the homogenized suspension what lead to obtain a chitosan hydrogel with a pH of 6.0-6.5, that is mixed with an agarose hydrogel, in a mass share ranging from 20 to 80% relative to the agarose hydrogel, wherein agarose hydrogel obtained from the polymer has a strength of 1.5% gel of at least 800 g/cm² and a gelation temperature < 40°C, and preferably has a low electrophoretic mobility value (EEO <0.15),wherein the agarose hydrogel is previously obtained by dissolving this polymer in distilled water at a temperature range of 60-80°C in an amount enabling to obtain a concentration of agarose in the range of 0.5 - 1.5% w/v and additional cell lines and/or cells for culture are added in an amount of 1-10% w/w relative to the composite.

4. Method according to claim 3, wherein the obtained composite, after mixing both hydrogels, is cooled to a temperature of 40-45°C, in which at this temperature it is in the form of a sol.

5. Method according to claim 3, wherein in order to obtain a gel form from sol, the temperature of the obtained composite is lowered, which causes a sol-gel phase transition during cooling with a temperature gradient of the phase transition from 5 to 39°C, preferably in the range of 5-39°C, most advantageously 28-36°C.

## Patentansprüche

1. Räumliche Objekte zur Verwendung in Zellkulturen und/oder mit Zelllinien, wobei die räumlichen Objekte durch Bioprinting einer Tinte in Form eines Hydrogelkomposits erhalten werden, **dadurch gekennzeichnet, dass** die Tinte während des Sol-Gel-Phasenübergangs in Form eines Sols oder eines Gels vorliegt, wobei das Komposit umfasst Wasser, Chitosan mit einem Massenanteil an Chitosan von 0,2-0,8 % w/w, vorzugsweise 0,4 % w/w, Agarose in einer Menge von 0,2-0,8 %, vorzugsweise 0,6 %, bezogen auf die Masse des vierkomponentigen Komposits, vorzugsweise 0,05 %, , und das Chitosan-Hydrogel einen pH-Wert von 0,6-6,5 aufweist, wobei das Polymer ein Molekulargewicht im Bereich von 50-375 kDa und einen Deacetylierungsgrad im Bereich von 60-95 %, vorzugsweise über 75 %, aufweist, und ein aus diesem Polymer erhaltenes Agarose-Hydrogel eine Gelstärke eines 1,5%igen Gels von mindestens 800 g/cm² und eine Gelierungstemperatur von <40 °C aufweist und vorzugsweise einen niedrigen Wert der elektrophoretischen Mobilität (EEO <0,15) aufweist, und das Komposit umfasst zusätzlich Zellen zur Kultivierung und/oder Zelllinien in Form einer Suspension in einem Verhältnis von 1-10 % w/w, bezogen auf die Masse des Komposits.

2. Räumliche Objekte zur Verwendung in Zellkulturen und/oder mit Zelllinien nach Ansprüche1, wobei die Tinte im Bereich von 40-45 °C ein Sol ist und ein Sol-Gel-Phasenübergang im Bereich von 5-39 °C, vorzugsweise 5-36 °C, erfolgt, wodurch die Erzeugung von Zellkulturen und/oder Zelllinien aus der Komposit-Bioprinting-Tinte ermöglicht wird.

3. Verfahren zur Herstellung von Zellkulturen und/oder Zelllinien, die aus einer Bioprinting-Tinte in Form eines Hydrogelkomposits auf Basis von Chitosan und Agarose erhalten werden, **dadurch gekennzeichnet, dass** das Verfahren umfasst den Schritt des anfänglichen Lösens von Chitosan in einer Lösung von Hydroxyoctansäure oder einer anderen flüchtigen organischen Säure, vorzugsweise mit einem pKa im Bereich von 3,0 bis 5,0, in einer Menge, die eine Konzentration dieses Polymers im Bereich von 1,0 % bis 2,5 % w/w, bezogen auf die Säurelösung, sicherstellt, wobei das Chitosan ein Molekulargewicht im Bereich von 50-375 kDa, vorzugsweise 180-300 kDa, und einen Deacetylierungsgrad im Bereich von 60-95 % aufweist, und wobei in dem nächsten Schritt Chitosan aus der zuvor hergestellten Lösung zu einem mikrokristallinen Niederschlag gefällt wird und der Chitosan-Niederschlag mit Wasser gewaschen wird, wobei das Wasser mindestens das Zweifache der Masse des vorläufig abgetrennten Niederschlags ausmacht, und danach der Niederschlag in destilliertem Wasser in einer Menge suspendiert wird, die das Erhalten einer Polymerkonzentration im Bereich von 0,5 % bis 1,5 % w/w, bezogen auf die Masse der Suspension, ermöglicht, und nach Homogenisierung einer Sättigung mit Kohlendioxid in einer Menge von 0,1-1 L/L, bezogen auf die homogenisierte Suspension, unterzogen wird, was zum Erhalten eines Chitosan-Hydrogels mit einem pH-Wert von 6,0-6,5 führt, das mit einem Agarose-Hydrogel in einem Massenanteil im Bereich von 20 bis 80 %, bezogen auf das Agarose-Hydrogel, gemischt wird, wobei das aus dem Polymer erhaltene Agarose-Hydrogel eine Gelstärke eines 1,5%igen Gels von mindestens 800 g/cm² und eine Gelierungstemperatur von <40 °C aufweist und vorzugsweise einen niedrigen Wert der elektrophoretischen Mobilität (EEO <0,15) aufweist, wobei das Agarose-Hydrogel zuvor durch Lösen dieses Polymers in destilliertem Wasser bei einer Temperatur im Bereich von 60-80 °C in einer Menge erhalten wird, die das Erhalten einer Agarosekonzentration im Bereich von 0,5-1,5 % w/v ermöglicht, und zusätzliche Zelllinien und/oder Zellen zur Kultivierung in einer Menge von 1-10 % w/w, bezogen auf das Komposit, zugegeben werden.

4. Verfahren nach Ansprüche 3, wobei das erhaltene Komposit nach dem Mischen beider Hydrogele auf eine Temperatur von 40-45 °C abgekühlt wird, bei welcher Temperatur es in Form eines Sols vorliegt.

5. Verfahren nach Ansprüche 3, wobei zum Erhalten einer Gelform aus einem Sol die Temperatur des erhaltenen Komposits abgesenkt wird, wodurch während des Abkühlens ein Sol-Gel-Phasenübergang verursacht wird, mit einem Phasenübergangstemperaturbereich von bis zu 39 °C, vorzugsweise im Bereich von 5-39 °C, am vorteilhaftesten 28-36 °C.

## Revendications

1. Objets spatiaux destinés à être utilisés dans des cultures cellulaires et/ou avec des lignées cellulaires, les objets spatiaux étant obtenus par bio-impression d'une encre sous la forme d'un composite d'hydrogel, **caractérisés en ce que** l'encre se présente sous la forme d'un sol ou d'un gel pendant la transition de phase sol-gel, dans lesquels le composite comprend de l'eau, du chitosane dans une fraction massique de chitosane de 0,2-0,8 % w/w, de préférence 0,4 % w/w, de l'agarose en une quantité de 0,2-0,8 %, de préférence 0,6 %, par rapport à la masse du composite à quatre composants, de préférence 0,05 %, et l'hydrogel de chitosane présente un pH de 0,6-6,5, dans lesquels le polymère a une masse moléculaire dans la gamme de 50-375 kDa et un degré de désacétylation de l'ordre de 60-95 %, de préférence supérieur à 75 %, et un hydrogel d'agarose obtenu à partir de ce polymère présente une force de gel de 1,5 % d'au moins 800 g/cm² et une température de gélification <40 °C, et présente de préférence une faible valeur de mobilité électrophorétique (EEO <0,15), et le composite comprend en outre des cellules destinées à la culture et/ou des lignées cellulaires sous la forme d'une suspension dans un rapport de 1-10 % w/w par rapport à la masse du composite.

2. Objets spatiaux destinés à être utilisés dans des cultures cellulaires et/ou avec des lignées cellulaires selon la revendication 1, dans lesquels l'encre est un sol d'environ 40-45 °C, et une transition de phase sol-gel se produisant dans la gamme de 5-39 °C, de préférence 5-36 °C, permettant la création de cultures cellulaires et/ou de lignées cellulaires à partir de l'encre de bio-impression composite.

3. Procédé de fabrication de cultures cellulaires et/ou de lignées cellulaires obtenues à partir d'une encre de bio-impression sous la forme d'un composite d'hydrogel à base de chitosane et d'agarose, **caractérisé en ce que** le procédé comprend l'étape de dissolution initiale du chitosane dans une solution d'acide hydroxyoctanoïque ou d'un autre acide organique volatil, de préférence ayant un pKa compris entre 3,0 et 5,0, en une quantité assurant une concentration de ce polymère dans la plage de 1,0 % à 2,5 % w/w par rapport à la solution acide, dans lequel le chitosane a une masse moléculaire dans la gamme de 50-375 kDa, de préférence 180-300 kDa, et un degré de désacétylation de l'ordre de 60-95 %, et, à l'étape suivante, le chitosane est précipité à partir de la solution préparée précédemment sous forme d'un dépôt microcristallin, et le dépôt de chitosane est lavé avec de l'eau, laquelle constitue au moins deux fois la masse du dépôt séparé préliminairement, et ensuite le dépôt est mis en suspension dans de l'eau distillée en une quantité permettant l'obtention d'une concentration de polymère d'environ 0,5 % à 1,5 % w/w par rapport à la masse de la suspension et, après homogénéisation, est soumis à une saturation avec du dioxyde de carbone en une quantité de 0,1-1 L/L par rapport à la suspension homogénéisée, ce qui conduit à l'obtention d'un hydrogel de chitosane ayant un pH de 6,0-6,5, qui est mélangé avec un hydrogel d'agarose dans une fraction massique allant de 20 à 80 % par rapport à l'hydrogel d'agarose, dans lequel l'hydrogel d'agarose obtenu à partir du polymère présente une force de gel de 1,5 % d'au moins 800 g/cm² et une température de gélification de <40 °C, et présente de préférence une faible valeur de mobilité électrophorétique (EEO <0,15), dans lequel l'hydrogel d'agarose est obtenu préalablement par dissolution de ce polymère dans de l'eau distillée à une température dans la plage de 60-80 °C en une quantité permettant l'obtention d'une concentration d'agarose dans la plage de 0,5-1,5 % w/v, et des lignées cellulaires supplémentaires et/ou des cellules destinées à la culture sont ajoutées en une quantité de 1-10 % w/w par rapport au composite.

4. Procédé selon la revendication 3, dans lequel le composite obtenu, après mélange des deux hydrogels, est refroidi à une température de 40-45 °C, température à laquelle il se présente sous la forme d'un sol.

5. Procédé selon la revendication 3, dans lequel, afin d'obtenir une forme de gel à partir d'un sol, la température du composite obtenu est abaissée, ce qui provoque une transition de phase sol-gel pendant le refroidissement, avec une plage de température de transition de phase allant jusqu'à 39 °C, de préférence dans la plage de 5-39 °C, de la manière la plus avantageuse 28-36 °C.
